# EUROPEAN PATENT APPLICATION

(11) **EP 3 428 142 A1**
(43) Date of publication of application: **16.01.2019**
(21) Application number: 18181814.7
(22) Date of filing: 05.07.2018
(51) Int. Cl.: C07C 7/144

(54) **PROCESS FOR SEPARATING PARAFFINS AND OLEFINS**

(30) Priority: 14.07.2017 EP 17181515
(71) Applicant: BASF SE, 67056 Ludwigshafen am Rhein (DE)
(72) Inventor: Benfer, Regina, 67056 Ludwigshafen (DE); Kappert, Emiel Jan, 67056 Ludwigshafen (DE); Kuhn, Jelan, 67056 Ludwigshafen (DE)
(74) Representative: BASF IP Association

(57) **Abstract**

The invention relates to a process for separating paraffins and olefins from a feed stream (5) comprising paraffins and olefins, wherein the paraffins and olefins to be separated all comprise the same number of carbon atoms, comprising following steps:
(a) extractive distillation of a stream comprising paraffins and olefins, thereby obtaining a stream enriched in paraffins (13) and a stream comprising olefins and extractant (23),
(b) distilling the second stream comprising olefins and extractant (23) for separating off the extractant, thereby obtaining a stream enriched in olefins (29),
wherein the stream enriched in olefins (29) is fed into a membrane separation (49) in which an essentially olefins comprising product stream (53) is obtained as permeate and a stream depleted in olefins is obtained as retentate (55) or
wherein before the extractive distillation a membrane separation (49) is carried out in which an essentially olefins comprising first product stream (53) is obtained as permeate and a stream depleted in olefins is obtained as retentate and the retentate is fed into the extractive distillation.

## Description

The invention relates to a process for separating paraffins and olefins from a feed stream comprising paraffins and olefins, wherein the paraffins and olefins to be separated all comprise the same number of carbon atoms, comprising the following steps:
(a) extractive distillation of a stream comprising paraffins and olefins, thereby obtaining a stream enriched in paraffins and a stream comprising olefins and extractant,
(b) distilling the second stream comprising olefins and extractant for separating off the extractant, thereby obtaining a stream enriched in olefins.

Paraffins and olefins result for example from thermal cracking of a petroleum fraction like liquidized petroleum gas, light gas or gas oil for the production of ethylene or propylene. Further, a stream comprising paraffins and olefins may result from a catalytic dehydrogenation or a fluid catalytic cracking process.

Due to small differences in relative fugacity of the components, the separation is a complex problem in distillative separation. Therefore, the separation of the components is carried out by an extractive distillation which means a distillation where a selective solvent also referred to as extractant is added. The extractant has a boiling point which is higher than the boiling point of the mixture to be separated. By adding the extractant, the relative fugacity of the component to be separated is increased.

For separation of butanes and butenes by extractive distillation using a selective extractant a number of processes is known. DE-A 102 19 375 for example discloses a continuous process for obtaining butenes from butanes, butenes and further C₃-C₅-hydrocarbons comprising mixture by extractive distillation with a selective solvent. The process is operated with an extractive distillation for separating butanes and a distillation of the bottom product for recycling the extractant and obtaining butenes.

Distillative or extractive processes for removing butanes to obtain butenes are disclosed for example in US-A 2005/0288471 or US 5,523,502.

Besides extractive and distillative processes it is also known to use membrane-based processes for separating olefins and paraffins. Such processes are disclosed for example in US-A 2006/0047176, J. Ploegmakers et al., "Economic Evaluation of Membrane Potential for Ethylene/Ethane Separation in a Retrofitted Hybrid Membrane-Distillation Plant Using Unisim-Design", Industrial and Engineering Chemistry Research, 2013, pages 6524-6539, or A. Motelica et al., "Membrane Retrofit Option for Paraffin/Olefin Separation - A technoeconomic Evaluation", Industrial and Engineering Chemistry Research, 2012, pages 6977-6986.

However, a particular disadvantage of extractive and distillation processes is the extremely high energy demand to achieve highly pure olefins. On the other hand, known processes using membranes only allow to obtain the olefins in high purity, but it is not possible to achieve high purities of both paraffins as well as olefins. The higher the demands on the purities of paraffins and olefins are, the higher is the energy consumption.

Therefore, it has been an object of the present invention to provide a process for separating paraffins and olefins from a feed stream comprising paraffins and olefins wherein the paraffins and olefins to be separated all comprise the same number of carbon atoms, and by which paraffins and olefins with high purity can be obtained.

This object is achieved by a process for separating paraffins and olefins from a feed stream comprising paraffins and olefins, wherein the paraffins and olefins to be separated all comprise the same number of carbon atoms, comprising the following steps:
(a) extractive distillation of a stream comprising paraffins and olefins, thereby obtaining a stream enriched in paraffins and a stream comprising olefins and extractant,
(b) distilling the second stream comprising olefins and extractant for separating off the extractant, thereby obtaining a stream enriched in olefins,
wherein the stream enriched in olefins is fed into a membrane separation in which an essentially olefins comprising product stream is obtained as permeate and a stream depleted in olefins is obtained as retentate or
wherein before carrying out the extractive distillation a membrane separation is carried out in which an essentially olefins comprising first product stream is obtained as permeate and a stream depleted in olefins is obtained as retentate and the retentate is fed into the extractive distillation.

By the inventive process two product streams are achieved, a first product stream containing olefins and a second product stream containing paraffins. The process further allows for achieving both product streams in high purity, which means that the amount of olefins in the olefin containing product stream is at least 50 % by weight, preferably 80 % to 99.8 % by weight and particularly 95 % to 99.5 % by weight and the amount of paraffins in the paraffin containing product stream is at least 50 % by weight, more preferred 80 % to 99.8 % by weight and particularly 95 % to 99.5 % by weight.

A further advantage of the process is that by the combination of extractive distillation and membrane separation processes, product streams with high purity can be achieved with much less energy consumption than by the well-known distillation processes. Particularly it is possible to use a stream enriched in olefins which is withdrawn from the distillation in step (b) which does not have the intended purity but a larger amount of impurities when the membrane separation process is carried out after the distillation. On the other hand, when carrying out the membrane separation process before the extractive distillation, the bottoms product of the extractive distillation in step (a) contains less olefins and therefore allows for a smaller distillation apparatus. Furthermore, it is also possible to only separate the extractant in the distillation in step (b) and to recycle the top stream of the distillation which is enriched in olefins into the membrane separation process which is carried out before feeding the stream containing olefins and paraffins into the extractive distillation.

The feed stream comprising paraffins and olefins for example is obtained in the production of ethylene and/or propylene by thermal or catalytic cracking of a petroleum fraction such as liquefied petroleum gas, light gas or gas oil. In an alternative, the paraffins and olefin containing feed stream can be obtained in the catalytic dehydrogenation. Besides paraffins and olefins, the feed stream comprising paraffins and olefins additionally may comprise small amounts of further hydrocarbons.

In a particularly preferred embodiment, the paraffins are butanes and the olefins are butenes. In this embodiment, the further hydrocarbons comprised in the feed stream are particularly further C₃- to C₅-hydrocarbons and may also comprise butynes, in particular 1-butyne (ethylacetylene) and butenyne (vinylacetylene). The butenes comprised in the stream are particularly n-butene, isobutene and 1,3-butadiene. The butanes are n-butanes or isobutane.

If the paraffins and olefins comprising feed stream emanates from an oil refinery or from a fluidized catalytic cracking plant, it generally has a composition of from 20 to 70 % by weight of paraffins, from 30 to 80 % by weight of olefins together with other hydrocarbons, in case of butanes and butenes as paraffins and olefins particularly C₃-C₅-hydrocarbons.

If the feed stream comprising paraffins and olefins is from a butadiene plant, the feed stream generally is the raffinate stream of the butadiene plant which comprises from 0 to 5 wt-% 1,3-butadiene, from 20 to 90 wt-% other butenes, from 10 to 80 wt-% butanes, from 0 to 3 wt-% other C₄-hydrocarbons and other hydrocarbons, in particular C₃- and C₅-hydrocarbons.

However, the paraffins and olefins comprising stream also may emanate from any other process in which such process streams are produced. Independent from the process the feed stream comprising paraffins and olefins comes from, the amount of paraffins preferably is in the range from 10 to 70 wt-%, in particular from 30 to 50 wt-% and the amount of olefins is in the range from 25 to 90 wt-% and in particular in the range from 50 to 70 wt-%. In each case, the balance generally are hydrocarbons with a different number of C-atoms, alkadienes or alkynes. If the paraffins and olefins are butanes and butenes, the butenes in the feed stream comprising paraffins and olefins generally comprise at least one of n-butenes, isobutenes and 1,3-butadiene. In most applications the butenes are a mixture of all types wherein the amount of each type of butene depends on the process where the feed stream emanates from.

The butanes in the feed stream comprising olefins and paraffins usually comprise n-butane and isobutanes, wherein the amounts of n-butane and isobutane also depend on the process the feed stream emanates from.

The extractive distillation can be carried out in any suitable apparatus for extractive distillations. A suitable apparatus for carrying out the extractive distillation particularly is a column. The column preferably comprises separation-active internals like trays, random packings or structured packings. Advantageously, the column has from 10 to 70 preferably from 20 to 50 and in particular from 30 to 40 theoretical stages.

The feed stream comprising paraffins and olefins can be fed into the apparatus for extractive distillation either in liquid or in gaseous form. Preferably, the feed stream is fed into the apparatus for extractive distillation in gaseous form. Besides the feed stream, an extractant is fed into the apparatus for an effective distillation. The extractant preferably is fed in liquid form.

When the extractive distillation is carried out in a column, this column may be separated in an upper section, a middle section and a lower section. The stream comprising olefins and paraffins preferably is fed between the middle and the lower section and the extractant is fed between the upper and the middle section of the column. On top of the column the reflux from the condenser is fed. The first stream enriched in paraffins is obtained at the top of the column and the second stream comprising olefins and extractant is obtained at the bottom. For supplying the heat which is necessary for the extractive distillation, it is preferred, to partly recycle the stream comprising olefins and extractant into the bottom of the column after being heated. To save energy, it is further preferred, to heat the partly recycled stream comprising olefins and extractant by transferring heat from the extractant obtained in the distillation in step (b). This heat transfer can be performed in any heat exchanger known to a skilled person.

The extractant used for the extractive distillation can be for example a selective solvent which affinity for hydrocarbons increases with the presence of double bonds and further with the presence of conjugated double bonds and triple bonds. Preferably, the extractant is a dipolar or polar, particularly preferably a dipolar aprotic, solvent. To simplify the choice of materials of construction for the apparatus, preference is given to materials which are non-corrosive or have a low corrosivity.

Extractants which are suitable for the process of the present invention are, for example, sulfolane, butyrolactone, nitriles such as acetonitrile, propionitrile, methoxypropionitrile, ketones such as acetone, furfural, N-alkyl-substituted lower aliphatic acid amides such as dimethylformamide, diethylformamide, dimethylacetamide, diethylacetamide, N-formylmorpholine, N-alkyl-substituted cyclic acid amides (lactams) such as N-alkylpyrrolidones, in particular N-methylpyrrolidone. In general, use is made of alkyl-substituted lower aliphatic acid amides or N-alkyl-substituted cyclic acid amides. Dimethylformamide, acetonitrile, furfural and especially N-methylpyrrolidone are particularly advantageous.

It is also possible to use mixtures of these solvents with one another, for example N-methylpyrrolidone with acetonitrile, mixtures of these solvents with cosolvents such as water and/or tert-butyl ethers, for example methyl tert-butyl ether, ethyl tert-butyl ether, propyl tert-butyl ether, n-butyl or isobutyl tert-butyl ether. Particularly when membranes incorporating silver or copper are used in the membrane separation, it is preferred to use mixtures of the solvent with water. Due to the fact that the streams in the extractive distillation usually already contain water, it is not necessary to add an additional device for removing the water.

A particularly useful selective solvent is N-methylpyrrolidone, in the present text referred to as NMP for short, preferably in aqueous solution, advantageously with from 0 to 20% by weight of water, in particular from 7 to 10% by weight of water, particularly preferably 8.3% by weight of water.

In a preferred embodiment, above the feed point for the extractant in the upper region of the column, there is preferable a backscrubbing zone comprising from 3-5 theoretical plates, in which residual extractant is scrubbed out by means of the runback condensed at the top of the column.

The pressure in the column for the extractive distillation is dependent on the temperature of the cooling medium and the condenser at the top of the column (well water, river water, seawater, refrigerant such as liquid propylene, liquid ammonia or brine). It is generally from 1 to 20 bar, frequently from 2 to 10 bar, preferably from 4 to 7 bar. The temperature in the column is, on the basis of the above-mentioned pressure values, set so as to give suitable thermodynamic conditions under which the selective solvent becomes laden with the components of the feed stream for which it has a greater affinity than for the paraffins while the paraffins in the stream remain in the gas phase. The temperature at the top of the column is typically in the range from about 30 to 60°C.

By these process conditions at the top of the column, a gaseous first stream enriched in paraffin is withdrawn. The amount of paraffins in the stream enriched in paraffins is in the range from 70 to 99.5 wt-%, preferably in the range from 90 to 99 wt-% and in particular in the range from 95 to 98 wt-%. Due to these high amounts of paraffins in the first stream enriched in paraffins, this stream can be withdrawn from the process as a paraffin product stream.

At the bottom of the column in which the extractive distillation is carried out, a liquid phase accrues which comprises the olefins and extractant. Additionally, this liquid comprises further components of the feed stream which are solved in the extractant.

The bottom stream comprising olefins and extractant is fed into the distillation in step (b). In the distillation in step (b), the stream comprising olefins and extractant is distilled to separate off the extractant. Thereby, a stream enriched in olefins is obtained. To separate the olefins and the extractant, the distillation preferably is carried out at a higher temperature and/or lower pressure than the extractive distillation. The distillation preferably is carried out in a distillation column, which may contain internals like trays, random packing or a structured packing. At the top of the distillation column, the stream enriched in olefins is withdrawn and at the bottom, the extractant. Besides remainders of the extractant, the stream enriched in olefins generally comprises traces of other hydrocarbons. The amount of olefins in the stream enriched in olefins generally is in the range from 60 to 99.5 wt-%, preferably in the range from 70 to 95 wt-% and in particular in the range from 75 to 85 wt-%.

The distillation column for separating the olefins and the extractant generally comprises from 4 to 20 theoretical stages, preferably from 6 to 15 theoretical stages and in particular from 8 to 12 theoretical stages. The pressure in the distillation column is in the range from 3 to 6 bar, in particular in the range from 4 to 5 bar. The bottom temperature in the distillation column depends on the pressure in the distillation column and the composition of the feed stream.

The two columns for the extractive distillation and the distillation of the extractant may also be combined to one column where a dividing wall is separating the upper part of the column. In this case the stream enriched in paraffins is drawn off on top at the side of the column, where the extractant is fed and the stream enriched in olefins is drawn off at the other top part.

To save energy, in an embodiment of the invention, a side stream is withdrawn from the distillation column, heated by heat transfer from the extractant obtained as bottom stream and fed back into the distillation column. By this heat transfer, heat of the distillation which else would be discharged with the extractant is returned to the distillation. Further, the extractant is cooled down, which allows recycling the extractant into the extractive distillation with less cooling which additionally has the advantage that less energy is needed for the cooling process.

As in processes which only comprise the extractive distillation of the feed stream comprising paraffins and olefins and the subsequent distillation of the bottoms stream of the extractive distillation the stream enriched in olefins still comprises extractant and hydrocarbons as impurities, according to the invention additionally a membrane separation process is carried out.

In a first embodiment, the stream enriched in olefins which is withdrawn from the distillation in step (b) is fed into the membrane separation process. The membrane used in the membrane separation process is selected such, that olefins pass the membrane and other components in the stream enriched in paraffins are retained. Thus, the permeate of the membrane separation process is obtained as the olefins comprising product stream. The amount of olefins in the olefins comprising product stream is in the range from 70 to 100 wt-%, more preferred in the range from 85 to 99.9 wt-% and in particular in the range from 95 to 99.5 wt-%. When the membranes produce a very pure olefin stream in the permeate and when such a high purity of the olefin stream is not required, part of the feed stream may be taken through a by-pass and mixed with the permeate stream, as to produce a olefin comprising product stream with the desired olefin purity.

As the retentate which is the stream depleted in olefins still may comprise olefins, in a preferred embodiment the retentate is recycled into the extractive distillation. This allows to separate the olefins from the retentate and thus to reduce the amount of valuable substances which is drained off.

In a second embodiment of the invention, the feed stream comprising paraffins and olefins is fed into the membrane separation process. In this embodiment, the retentate of the membrane separation process is fed into the extractive distillation. By the membrane separation process olefins are separated off the feed stream. Therefore, the feed stream fed into the extractive distillation comprises a reduced amount of olefins. In this embodiment, the stream enriched in olefins which is withdrawn from the distillation in step (b) also can be discharged as a product stream. Should the amount of impurities be too large, it is also possible to recycle the stream enriched in olefins. In this case, the stream enriched in olefins preferably is recycled into the membrane separation process.

In both embodiments, the membrane used in the membrane separation process is a membrane which is selective for olefins. This means, that the membrane can be passed by the olefins and the paraffins and other components in the stream fed into the membrane separation process are retained and leave the membrane separation process as retentate. Due to the membrane used in the membrane separation process, the permeate can be withdrawn as olefin comprising product stream.

It is preferred, to only perform one membrane separation process, either following the extractive distillation or before carrying out the extractive distillation. It is, however, also possible to carry out two membrane separation processes, one according to the first embodiment after the extractive distillation and a second according to the second embodiment before the extractive distillation.

The membrane used in the membrane separation process can be any membrane known to a person skilled in the art which allows selective permeation of olefins. This membrane separation can, for instance, take place based on affinity or molecular size. The separation layer of such membranes could be based for example on organic or inorganic materials, including zeolites, metallo-organic frameworks (MOFs), a mixed-matrix membrane, carbon molecular sieve, polymers, thermally-rearranged polymers, polymers of intrinsic microporosity (PIM), inorganic-organic hybrids, or a (supported) ionic liquid. Suitable membranes particularly are membranes containing silver or copper ions. The olefins reversely bind to the silver or copper ions and are thus transported through the membrane. Suitable membranes are disclosed for example in US-A 2004/215045, or US-A 2015/025293.Typically, these membranes need to be continuously humidified, since they show a significant performance loss if applied in dry streams. For this reason, the application of these membranes in combination with an extractive distillation for which a solvent-water mixture is used is of particular interest, as the olefins come out of the distillation columns saturated with water, which is desired for the membrane separation.

Independent of the position of the membrane separation process, it is possible to operate the membrane separation process in gas separation mode, in vapor permeation mode or in pervaporation mode. In gas separation mode or in vapor permeation mode, the stream fed into the membrane separation process, the retentate and the permeate are all in the gas phase. In pervaporation mode, the stream fed into the membrane separation process and the retentate are liquid and the permeate is in the gas phase. In the pervaporation mode the stream fed into the membrane separation process and/or the stream fed into the membrane modules generally are heated.

For practical reasons, presently used membrane separation processes for separating olefins and paraffins are operated in gas separation mode. To achieve a sufficient separation of olefins from the retentate, it is preferred, to compress the stream enriched in olefins before feeding it into the membrane separation process. By this compression it is possible to increase the pressure difference between the retentate and the permeate and to improve the separating capacity in this way. To further enhance the flux through the membranes and especially to further deplete the retentate stream of olefins, a vacuum, particularly at pressure from 50 to 500 mbar (abs) can be drawn at the permeate side of the membranes. Alternatively, a sweep gas or steam sparging could be used to decrease the olefin partial pressure on the permeate side.

On the other hand, if the membrane separation process is carried out before the extractive distillation, it is also preferred to compress the feed stream containing olefins and paraffins for the same reasons before feeding it into the membrane separation process to improve the separating capacity.

The membrane separation unit used in the membrane separation process may comprise additional units like a humidification unit, a hydrogenation unit or an S-removal unit. Such units generally are used to achieve a feed stream into the membrane separation unit which does not impair the membrane.

To remove low boilers which may be comprised in the stream enriched in olefins which is withdrawn from the distillation in step (b), it is preferred to cool down this stream enriched in olefins to a temperature below the boiling point of the olefins and to feed it into a phase separator in which remaining gases and the liquefied olefins are separated and the remaining gases are withdrawn as off-gas. The cooled stream from which the low boilers are removed is then fed into the membrane separation process.

As there may be undesired high boilers in the feed stream, it is preferred to remove the undesired high boilers by taking off a small purge stream from the extractant which is obtained at the bottom of the distillation column and recycled into the extractive distillation. It is further possible to take of a part stream from the extractant, feed this stream into a treatment unit where high boilers are removed from the stream and withdraw a purge stream containing the high boilers from the process. The rest of the stream then is fed back into the extractant stream which is recycled into the extractive distillation.

In order to save energy, it is further preferred, to use heat from hot streams which have to be cooled down for heating streams which have to be heated. To use process heat in such a way, it is for example possible to cool the extractant obtained in step (b) in a heat exchanger by transferring heat to the feed stream. Depending on the amount of extractant and the amount of the feed stream, the temperature difference between the two streams and the temperature to which the feed stream should be heated, it is either possible to obtain the full heating by this heat transfer or only a part of the heating. If only a part of the heating can be achieved, it is necessary to further heat the feed stream to the desired temperature in any heating device known to a skilled person. Preferred heating devices are heat exchangers. In this case the heating medium for example is superheated steam or any other heating medium like thermal oil or water. The heating medium thereby depends on the temperature to which the feed stream is to be heated.

As the feed stream preferably is fed into the extractive distillation in gas phase, the feed stream has to be heated to a temperature above the boiling temperature of the mixture contained in the feed stream. The temperature to which the feed stream is heated depends on the pressure of this stream.

If a membrane separation is carried out before feeding the feed stream into the extractive distillation, it is preferred to heat the feed stream being fed into the membrane separation process. As it is preferred to carry out the membrane separation at elevated pressure, the temperature to which the feed stream is heated before being fed into the membrane separation process is in such range that the feed is in the vapor state. Alternatively, the process can be run in pervaporation mode, where the feed is in the liquid state, but where higher temperatures lead to an increased driving force over the membrane. In principle, the process benefits from higher temperatures, but the temperature is limited by the stability of the membranes and the operationability of the membrane plant. For typical polymeric membranes, it is preferred to operate the process up to temperatures of 80 °C, more preferably up to 60 °C. For ceramic, carbonic, zeolitic, MOF, or high-performance polymeric membranes, temperatures up to 300 °C can theoretically be applied.

The pressure of the feed stream being fed into the membrane separation preferably is in the range from 1 to 30 bar, more preferred in the range from 3 to 15 bar and particularly in the range from 5 to 12 bar. As the membrane separation preferably is carried out with a pressure of the stream fed into the membrane separation which is higher than the preferred pressure in the extractive distillation, it is further preferred, to expand the retentate before feeding into the extractive distillation. The pressure to which the retentate is expanded preferably corresponds to the pressure at which the extractive distillation is carried out when no membrane separation is operated before.

As it cannot be excluded that the product stream comprising essentially olefins which is withdrawn from the membrane separation process still contains low boilers, it is further preferred, to cool down the product stream comprising essentially olefins to a temperature below the boiling point of the olefins comprised in the product stream and to feed the cooled stream into a phase separator in which remaining gases and the liquefied olefins are separated and the remaining gases are withdrawn as off-gas. After separation of the low boilers, the liquefied olefins only contain traces of impurities and can be withdrawn as product stream. The amount of the different olefins comprised in the product stream depends on the composition of the feed stream.

Embodiments of the invention are shown in the figures and described in more detail in the following description.

In the figures:
- Figure 1: shows a flow chart of a separation process of paraffins and olefins according to the state of the art,
- Figure 2: shows a flow chart of a separation process of paraffins and olefins with a membrane separation following the extractive distillation in a first embodiment,
- Figure 3: shows a flow chart of a separation process of paraffins and olefins with a membrane separation following the extractive distillation in a second embodiment,
- Figure 4: shows a flow chart of a separation process of paraffins and olefins with a membrane separation following the extractive distillation in a third embodiment,
- Figure 5: shows a flow chart of a separation process of paraffins and olefins with a membrane separation following the extractive distillation in a forth embodiment,
- Figure 6: shows a flow chart of a separation process of paraffins and olefins with a membrane separation following the extractive distillation in a fifth embodiment,
- Figure 7: shows a flow chart of a separation process of paraffins and olefins with a membrane separation following the extractive distillation in a sixth embodiment,
- Figure 8: shows a flow chart of a separation process of paraffins and olefins with a membrane separation following the extractive distillation in a seventh embodiment,
- Figure 9: shows a flow chart of a separation process of paraffins and olefins with a membrane separation before operating the extractive distillation.

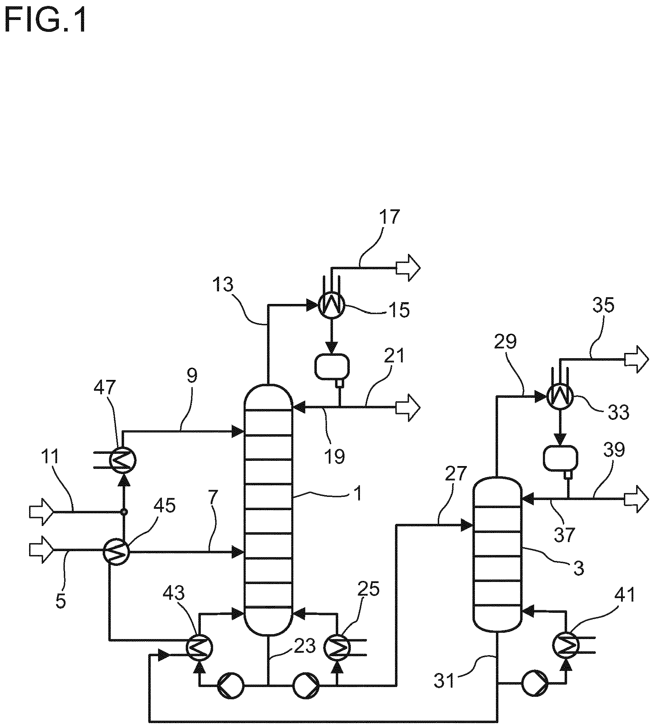

Figure 1 shows a separation process of a stream comprising paraffins and olefins by extractive distillation according to the state of the art.

The device for separating paraffins and olefins, in particular for separating butanes and butenes, comprises a first column 1 and a second column 2. Into the first column 1, a feed stream 5, comprising paraffins and olefins is fed via a side inlet 7. The feed stream preferably is gaseous.

Via a second side inlet 9, which is above the side inlet 7 for the feed stream, a liquid extractant is fed into the first column 1. Additional extractant may be added via an extractant feed stream 11.

In the first column, an extractive distillation is carried out. At the top of the first column 1, a stream 13 enriched in paraffins is withdrawn. The stream 13 enriched in paraffins is cooled in a heat exchanger 15 below the boiling point of the paraffins. Low boilers which have a boiling point below the boiling point of the paraffins are withdrawn as offgas 17. The remaining, liquid paraffins comprising stream is divided into a first liquid paraffins comprising stream 19 which is recycled into the first column 1 and a second liquid paraffins comprising stream 21 which is withdrawn as a paraffins containing product stream.

At the bottom of the first column 1 a stream 23 comprising olefins and extractant is withdrawn. A part of the stream 23 comprising olefins and extractant is heated in a heat exchanger 25 and recycled into the first column 1. A further part of the stream 23 comprising olefins and extractant is fed into the second column 3 via an inlet 27. In the second column 3, a distillation of the stream takes place separating the stream into the stream 29 enriched in olefins and a bottom stream 31 containing extractant.

The stream 29 enriched in olefins is fed into a heat exchanger 33 for cooling the stream 29 below the boiling point of the olefins in order to remove low boilers. The low boilers which not have been condensed in the heat exchanger 33 are removed as offgas 35. The liquid stream is divided into two parts, a first part 37 of the liquid stream being recycled into the second column 3 and the second part of the liquefied olefins containing stream is withdrawn as an olefin containing product stream 39.

A part of the bottom stream 31 containing extractant is heated in a heat exchanger 41 and recycled back into the second column 3. For heat recovery, the rest of the bottom stream 31 containing extractant flows through a first heat exchanger for heat recovery 43 and a second heat exchanger for heat recovery 45. In the first heat exchanger for heat recovery 43 heat is transferred to a third part of the stream 23 comprising olefins and extractant. This third part of the stream 23 comprising olefins and extractant is recycled back into the first column 1.

The second heat exchanger for heat recovery 45 is used to heat the feed stream 5. As it is preferred that the feed stream 5 is fed into the first column 1 in a gaseous form, it is either possible to increase the temperature of an already gaseous feed stream 5 or to evaporate the feed stream 5 in the second heat exchanger for heat recovery 45.

By heating the third part of the stream 23 comprising olefins and extractant in the first heat exchanger for heat recovery 43 and by heating the feed stream 5 in the second heat exchanger for heat recovery 45 by heat transfer from the extractant comprising stream 31, simultaneously, the extractant comprising stream is cooled.

As it is further preferred that the extractant is fed into the first column 1 with a defined temperature, an additional heat exchanger 47 is provided in the bottom stream 31 containing extractant after passing the stream through the first heat exchanger for heat recovery 43 and the second heat exchanger for heat recovery 45 to cool this stream to a temperature with which the extractant shall be fed into the first column 1 via the second side inlet 9.

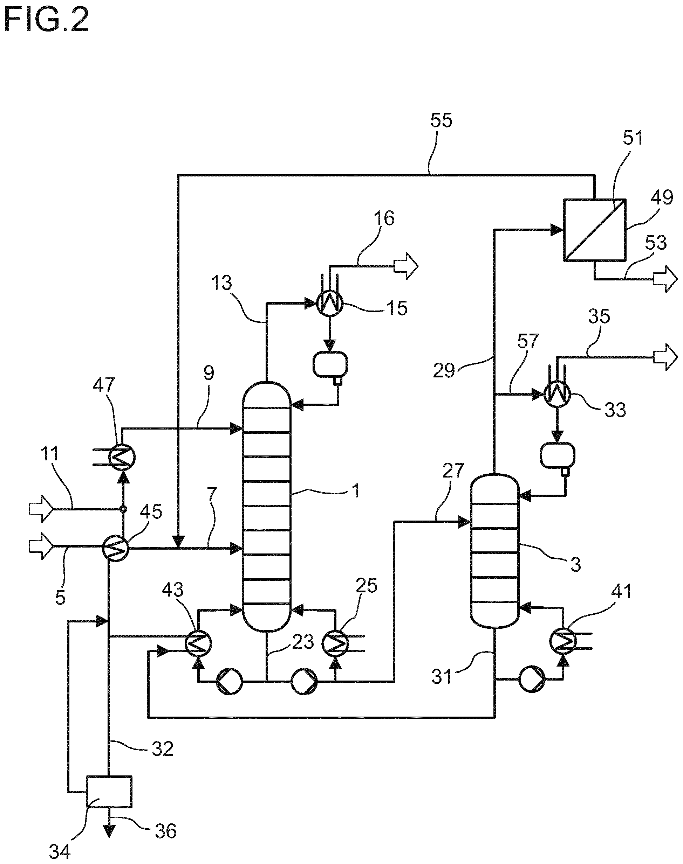

Figure 2 shows a flow chart of a separation process of paraffins and olefins with a membrane separation following the extractive distillation in a first embodiment.

In the embodiment shown in Figure 2, the extractive distillation part corresponds to that of the state of the art. As it is very energy intensive to obtain an essential olefin containing product stream by a distillation process, according to the invention, an additional membrane separation is carried out.

According to the first embodiment of the invention, a membrane unit 49 is provided. The stream 29 enriched in olefins is fed into the membrane unit 49. The membrane unit 49 comprises a membrane 51 which allows olefins to pass. In the membrane unit 49, olefins therefore pass the membrane 51 and an essentially olefins comprising product stream 53 is obtained as permeate. A stream depleted in olefins is withdrawn from the membrane unit 49 as retentate 55. The retentate 55 is recycled and mixed with the feed stream 5 which is fed into the first column 1 via the side inlet 7. A part stream 57 of the stream enriched in olefins 29 is branched off and fed into the heat exchanger 33 in which this part stream 57 is cooled to a temperature below the boiling point of the olefins. The condensed olefins are recycled into the second column 3 and non-condensed low boilers are withdrawn as offgas 35.

Further, the stream enriched in paraffins 13 which is withdrawn at the top of the first column 1 is cooled in the heat exchanger 15 and a part of this stream is withdrawn as a gaseous product stream 16 and the condensed stream is recycled back into the first column 1.

To remove undesired high boilers, a part 32 of the bottom stream 31 containing extractant can be removed as a purge stream. However, it is preferred to feed the part 32 of the bottom stream 31 into a treatment unit 34, where high boilers are removed from the bottom stream 31 containing extractant and withdrawn from the process as purge stream 36. The remaining stream containing extractant depleted of high boilers is fed back into the bottom stream 31 containing extractant and recycled into the first column 1. The treatment unit 34 can be for example a distillation. Depending on the feed composition, further processes to remove high boilers can be precipitation, membrane processes or other separation units.

The part 32 of the bottom stream 31 containing extractant can be removed at any point from the bottom stream 31 containing extractant after leaving the second column 3 and before entering the first column 1.

The removal of high boilers which is exemplary shown in figure 2 can also be used in all embodiments shown in the following figures.

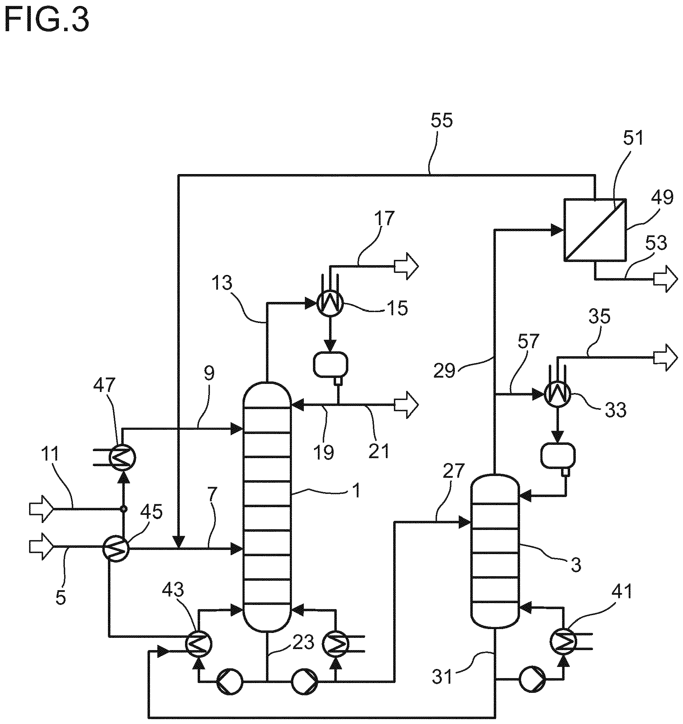

A second embodiment of the inventive process for separating paraffins and olefins is shown in Figure 3.

The embodiment shown in Figure 3 differs from the embodiment of Figure 2 in the treatment of the stream 13 enriched in paraffins.

In the embodiment as shown in Figure 3, the stream enriched in paraffins 13 is treated as in the extractive distillation of the state of the art as shown in Figure 1 by cooling the stream enriched in paraffins 13 in the heat exchanger 15 to a temperature below the boiling point of the paraffins, wherein low boilers which are not condensed in the heat exchanger 15 are withdrawn as offgas 17, part of the condensed paraffins is recycled into the first column 1 as first liquid paraffins comprising stream 19 and a second liquid paraffins comprising stream 21 is withdrawn as product stream.

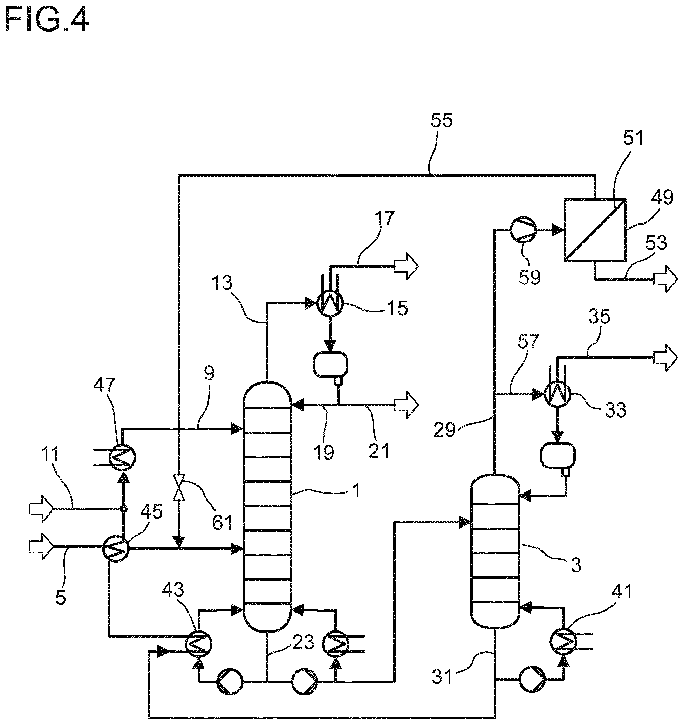

A third embodiment of the invention is shown in Figure 4. The process of Figure 4 widely corresponds to that of the second embodiment shown in Figure 3. In difference to the process as shown in Figure 3, in the process of the third embodiment in Figure 4, the stream 29 enriched in olefins is compressed in a compressor 59 before being fed into the membrane unit 49. By increasing the pressure in the compressor 59, the performance of the membrane unit 49 can be increased. As in this case, the retentate 15 has a pressure which is higher than the pressure of the feed stream 5, a throttle unit 61, for example a valve or any other throttle or means for reducing pressure, is integrated in the line in which the retentate 55 is recycled into the feed stream 5. In the throttle unit 61, the elevated pressure of the retentate 55 which is withdrawn from the membrane unit 49 is reduced to the pressure for the extractive distillation carried out in the first column 1.

The compression of the stream enriched in olefins 29 and the decompression of the retentate before recycling into the first column 1 also can be performed in all other embodiments as shown in Figures 2, 3 and 5 to 7.

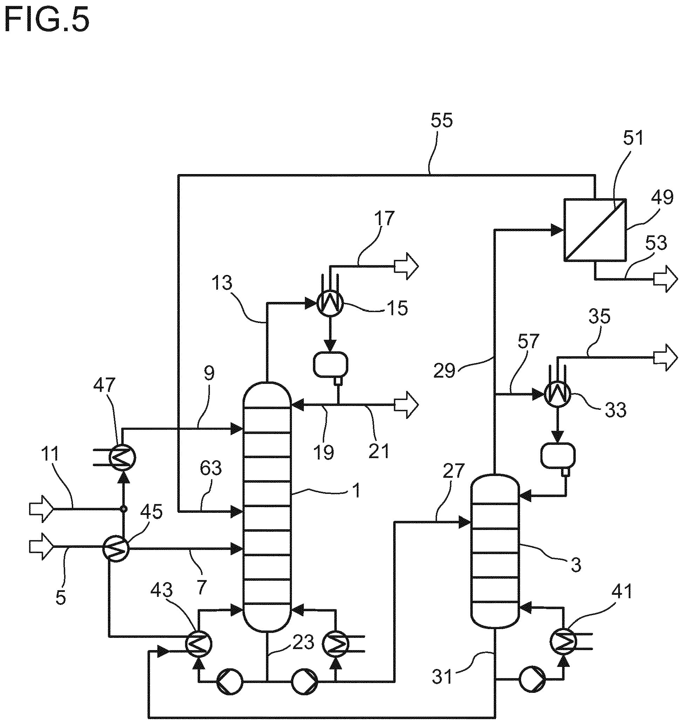

In the embodiment as shown in Figure 5, in difference to the previous embodiments the retentate stream 55 is not combined with the feed stream 5 but fed into the first column 1 via an additional inlet 63. The additional inlet 63 in this embodiment is above the side inlet 7 through which the feed stream is introduced into the first column 1. Further, the additional inlet 63 is below the second side inlet 9 through which the extractant is fed into the first column 1. However, besides the embodiment as shown in figure 5 with the additional side inlet 63 being positioned above the side inlet 7 through which the feed stream is introduced it is also possible to provide the additional side inlet 63 below the side inlet 7.

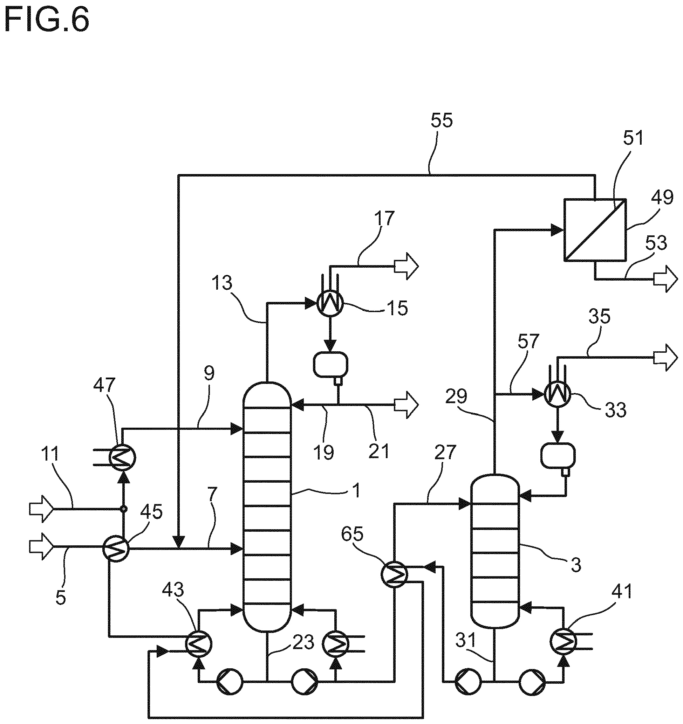

A fifth embodiment of the inventive process is shown in Figure 6. In the embodiment as shown in Figure 6, an additional third heat exchanger for heat recovery 65 is used. The third heat exchanger for heat recovery 65 is placed in the stream comprising olefins and extractant which is fed into the second distillation column 3. In the third heat exchanger for heat recovery 65 the stream comprising olefins and extractant is heated before being fed into the second column 3. For this purpose, the part of the bottom stream 31 containing extractant which is recycled into the first column 1 is used. In the third heat exchanger for heat recovery 65, the bottom stream 31 containing extractant transfers heat to the stream comprising olefins and extractant and thereby, the bottom stream containing extractant is cooled and the stream comprising olefins and extractant which is fed into the second column 3 is heated.

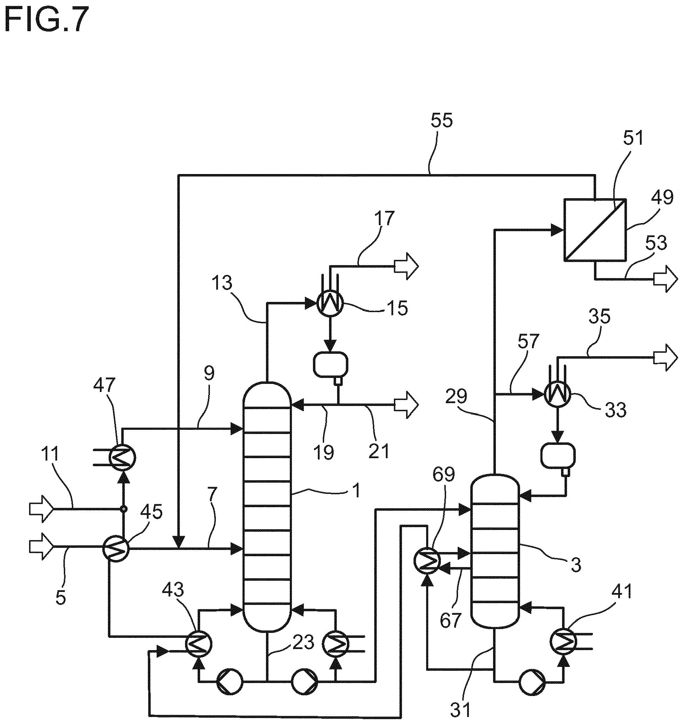

A further option for heat recovery is shown in the sixth embodiment in Figure 7.

In the embodiment as shown in Figure 7, a side stream 67 is withdrawn from the second column 3 fed into a heat exchanger 69 in which heat is transferred from the bottom stream 31 containing extractant to the side stream 67. By this heat transfer, the bottom stream 31 containing extractant is cooled and the side stream 67 is heated. After passing the heat exchanger 69, the side stream 67 is recycled into the second column 3. The inlet for recycling the side stream 67 into the second column 3 can be placed on the same tray or the same height as the outlet for the side stream 67 or below or above. Preferred is a return of the side stream on the same tray or at the same height as the outlet of the side stream or below the outlet of the side stream. As part of the stream 67 coming from the heat exchanger 69 may be vaporized the inlet stream may also be divided into a liquid part and a vaporous part which may be introduced to the column on different points.

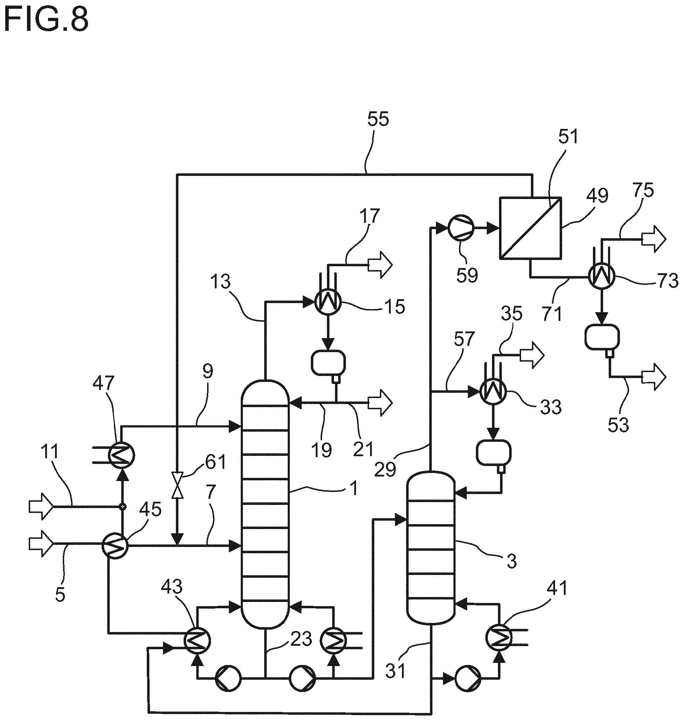

In Figure 8, a seventh embodiment of the inventive process is shown. The process thereby corresponds to that of Figure 4, however, an additional low boiler separation is provided following the membrane separation. For this low boiler separation, the permeate 71 leaving the membrane unit 49 is fed into a heat exchanger 73 in which the permeate is cooled to a temperature below the boiling point of the olefins. Due to this cooling, the olefins condense and low boilers remain in gaseous form. The low boilers are removed from the process as offgas 75 and the condensed olefins are removed as the essentially olefins comprising product stream 53.

Depending on the low boilers content in the essentially olefins comprising product stream 53 of the embodiments as shown in Figures 2 to 7, it is also possible to additionally carry out the low boilers removal from the permeate of the embodiment in Figure 8 in these embodiments.

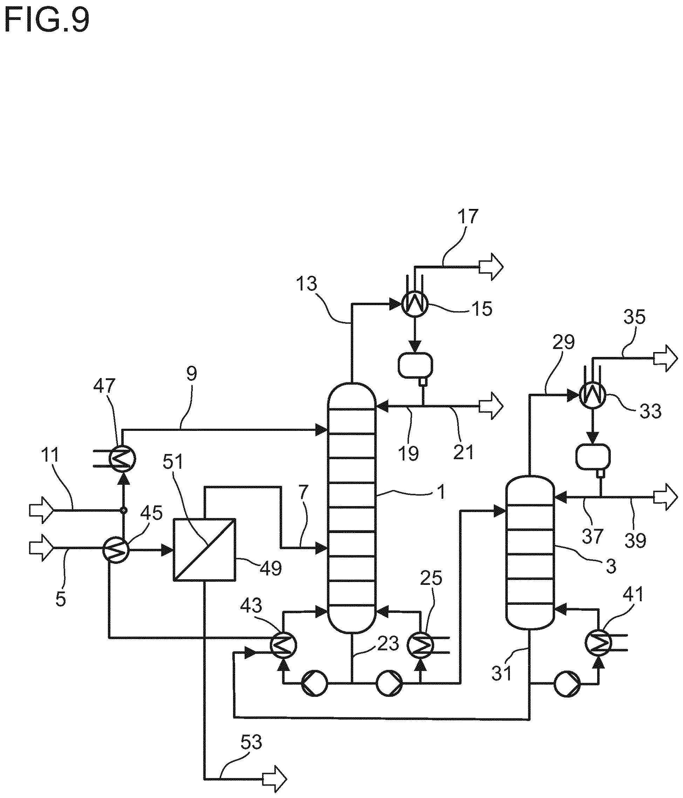

Finally, as an alternative, a separation process with a membrane separation before operating the extractive distillation as an alternative is shown in Figure 9.

In difference to the embodiments as shown in Figures 2 to 8, in the embodiment shown in Figure 9, the membrane unit 49 is incorporated in the feed stream 5 behind the second heat exchanger for heat recovery 45. In the membrane unit 49 olefins are separated from the feed stream 5 by passing the membrane 51. The retentate being depleted in olefins is fed into the first column 1 via the inlet 7. The permeate is withdrawn from the membrane unit 49 as essentially olefins comprising product stream 53.

In all embodiments shown, the membrane separation preferably is operated in gas separation mode which means that the stream fed into the membrane unit is in gas form and also the retentate and permeate are gaseous. The membrane 51 used in the membrane unit 49 is - as described above - a membrane selective for olefins. Such a membrane preferably is a membrane containing silver or copper ions. However, it is also possible to operate the membrane separation in pervaporation mode. In this case the stream enriched in olefins 29 or the feed stream 5 is fed into the membrane as a liquid and heated in the membrane unit 49 such that it at least partly evaporates. The permeate therefore is gaseous.

To enhance the flux through the membranes and particularly to further deplete the retentate stream of olefins it is possible to apply a vacuum at the permeate side of the membrane unit 49. Preferably, the pressure on the permeate side is in the range from 50 to 500 mbar(abs). As an alternative to the vacuum it is also possible to use a sweep gas or steam sparging to decrease the partial pressure of the olefins on the permeate side of the membrane unit 49.

Besides the processes as shown in Figures 2 to 9 containing only one membrane separation process, it is also possible to combine the embodiment of Figure 9 with a membrane separation in the feed stream with an additional membrane separation of the olefin enriched stream withdrawn at the top of the second column 3. These two membrane separation processes particularly are suitable if in case the amount of olefins in the feed stream is quite high and further, the amount of olefins in the stream enriched in olefins 29 is too low to fulfil the desired specification.

In contrast to the process of the art as shown in Figure 1, it is possible to operate the extractive distillation with a smaller amount of extractant and/or less energy consumption as the additional membrane separation process allows for a higher amount of paraffins in the stream enriched in olefins and thus the extractive distillation can be operated in such a way that a larger amount of paraffins remains in the bottoms stream of the extractive distillation.

In all embodiments, pumps are shown for symbolizing the transport of the respective streams. However, in some embodiments, where the flux of the stream can be realized in another way, it may be possible to omit the pumps in this stream.

**List of Reference Numbers**

| | | | |
|---|---|---|---|
| 1 | first column | 63 | additional inlet |
| 3 | second column | 65 | third heat exchanger for heat recovery |
| 5 | feed stream | 67 | side stream |
| 7 | side inlet | 69 | heat exchanger |
| 9 | second side inlet | 71 | permeate |
| 11 | extractant feed stream | 73 | heat exchanger |
| 13 | stream enriched in paraffins | 75 | offgas |
| 15 | heat exchanger | | |
| 16 | gaseous product stream | | |
| 17 | offgas | | |
| 19 | first liquid paraffins comprising stream | | |
| 21 | second liquid paraffins comprising stream | | |
| 23 | stream comprising olefins and extractant | | |
| 25 | heat exchanger | | |
| 27 | inlet | | |
| 29 | stream enriched in olefins | | |
| 31 | bottom stream containing extractant | | |
| 32 | part of the bottom stream 31 containing extractant | | |
| 33 | heat exchanger | | |
| 34 | treatment unit | | |
| 35 | offgas | | |
| 36 | purge stream | | |
| 37 | first part of the liquid stream | | |
| 39 | olefin containing product stream | | |
| 41 | heat exchanger | | |
| 43 | first heat exchanger for heat recovery | | |
| 45 | second heat exchanger for heat recovery | | |
| 47 | heat exchanger | | |
| 49 | membrane unit | | |
| 51 | membrane | | |
| 53 | essentially olefins comprising product stream | | |
| 55 | retentate | | |
| 57 | part stream | | |
| 59 | compressor | | |
| 61 | throttle unit | | |

## Claims

1. A process for separating paraffins and olefins from a feed stream (5) comprising paraffins and olefins, wherein the paraffins and olefins to be separated all comprise the same number of carbon atoms, comprising following steps:
(a) extractive distillation of a stream comprising paraffins and olefins, thereby obtaining a stream enriched in paraffins (13) and a stream comprising olefins and extractant (23),
(b) distilling the second stream comprising olefins and extractant (23) for separating off the extractant, thereby obtaining a stream enriched in olefins (29),
wherein the stream enriched in olefins (29) is fed into a membrane separation (49) in which an essentially olefins comprising product stream (53) is obtained as permeate and a stream depleted in olefins is obtained as retentate (55) or
wherein before the extractive distillation a membrane separation (49) is carried out in which an essentially olefins comprising first product stream (53) is obtained as permeate and a stream depleted in olefins is obtained as retentate and the retentate is fed into the extractive distillation.

2. The process according to claim 1, wherein when the stream enriched in olefins (29) is fed into the membrane separation the retentate (55) is recycled back into the extractive distillation.

3. The process according to claim 1 or 2, wherein the stream enriched in olefins (29) is compressed before being fed into the membrane separation (49) and/or a vacuum is applied at the permeate side of the membrane separation unit (49).

4. The process according to any of claims 1 to 3, wherein the membrane (51) used in the membrane separation process is a membrane incorporating silver or copper.

5. The process according to any of claims 1 to 4, wherein the extractant comprises N-methyl pyrrolidone, dimethylformamide, acetonitrile, furfural and mixtures thereof and mixtures of water and one or more of N-methyl pyrrolidone, dimethylformamide, acetonitrile and furfural.

6. The process according to any of claims 1 to 5, wherein the stream enriched in olefins (29) and obtained in step (b) is cooled down to a temperature below the boiling point of the olefins and is fed into a phase separator in which remaining gases and the liquefied olefins are separated and the remaining gases are withdrawn as off-gas.

7. The process according to any of claims 1 to 6, wherein the extractant obtained in step (b) is cooled in a heat exchanger (43) by transferring heat to the feed stream (5).

8. The process according to any of claims 1 to 7, wherein the extractant obtained in step (b) is compressed and then cooled in a heat exchanger (65) by transferring heat to the stream comprising olefins and extractant (23).

9. The process according to any of claims 1 to 8, wherein the product stream comprising essentially olefins (53) is cooled down to a temperature below the boiling point of the olefins comprised in the product stream and fed into a phase separator in which remaining gases and the liquefied olefins are separated and the remaining gases are withdrawn as off-gas.

10. The process according to any of claims 1 to 9, wherein the extractive distillation is carried out in a column (1) into which the stream (5) comprising olefins and paraffins is fed as a side stream, the extractant is fed at the top of the column (1), the first stream enriched in paraffins (13) is obtained at the top of the column (1) and the second stream comprising olefins and extractant (23) is obtained at the bottom.

11. The process according to claim 10, wherein the stream comprising olefins and extractant (23) is partly recycled into the bottom of the column (1) after being heated.

12. The process according to any of claims 1 to 11, wherein the distillation in step (b) is carried out in a distillation column (3) and the extractant is obtained as bottom stream (31) and the stream enriched in olefins (29) as top stream.

13. The process according to claim 12, wherein a side stream (67) is withdrawn from the distillation column (3), heated by heat transfer from the extractant obtained as bottom stream and fed back into the distillation column.

14. The process according to any of claims 1 to 13, wherein the extractive distillation in step (a) and the distillation in step (b) are carried out in one column where a dividing wall is separating the upper part of the column.

15. The process according to any of claims 1 to 14, wherein the paraffins are butanes and the olefins are butenes.
